**Europäisches Patentamt**

**European Patent Office**

**Office européen des brevets**

(19)

(11) Numéro de publication: **0 089 600**
**B2**

(12) **NOUVEAU FASCICULE DE BREVET EUROPEEN**

(45) Date de publication du nouveau fascicule du brevet: **16.01.91**

(51) Int. Cl.⁵: **F 01 D 9/04,** F 01 D 5/14

(21) Numéro de dépôt: **83102530.9**

(22) Date de dépôt: **15.03.83**

(54) **Aubage directeur pour veines divergentes de turbine à vapeur.**

(30) Priorité: **19.03.82 FR 8204687**

(43) Date de publication de la demande:
**28.09.83 Bulletin 83/39**

(45) Mention de la délivrance du brevet:
**29.01.86 Bulletin 86/05**

(45) Mention de la decision concernant l'opposition:
**16.01.91 Bulletin 91/03**

(84) Etats contractants désignés:
**AT BE CH DE FR GB IT LI LU NL SE**

(56) Documents cités:
| | |
|---|---|
| EP-A-0 064 679 | GB-A-1 080 015 |
| CH-A- 229 924 | GB-A-1 116 580 |
| FR-A- 892 412 | US-A-1 389 910 |
| FR-A-1 037 610 | US-A-1 475 212 |
| FR-A-2 003 356 | US-A-3 529 631 |
| GB-A- 235 180 | |

**Walter Traupel "Thermische Turbomaschinen",
3ième édition, 1ière partie, 1977, Springer-
Verlag, page 414;**

**Dejc et Trojanovskij "Untersuchung und
Berechnung axialer Turbinenstufen", 1973, VEB
Verlag Technik, Berlin, pages 489 et 491**

(73) Titulaire: **GEC ALSTHOM SA
38, avenue Kléber
F-75116 Paris (FR)**

(72) Inventeur: **Bessay, Raymond
8, rue de Stockholm
F-90000 Belfort (FR)**
Inventeur: **Riollet, Gilbert
11, rue du Cherche Midi
F-75006 Paris (FR)**

(74) Mandataire: **Weinmiller, Jürgen et al
Lennéstrasse 9 Postfach 24
D-8133 Feldafing (DE)**

(56) Documents cités:
**Journal of Engineering for Power, janvier 1972
Harmon et al,"Performance of Compressor
Blade Rows in a Sloping Flowpath", pages 61-69**

**Description**

La présente invention a trait a un aubage directeur pour veines divergentes de turbine à vapeur comportant des aubes disposées entre un plancher parallèle à l'axe 0 0' de la turbine et un plafond et dont l'extrados et l'intrados sont constitués par des génératrices sensiblement rectilignes, l'angale de divergence $\theta$ de la veine étant comprise entre 25° et 70°.

Dans l'ouvrage de Dejc et Trojanvskij "Untersuchung und Berechnung axialer Turbinenstufen", VEB Verlag Technik, Berlin 1973, pages 489 à 491, il est décrit (figure 8—11) un aubage directeur avec un plancher parallèle à l'axe (vp = 0) et un angle de divergence de la veine égal à 30° et des génératrices orthogonales au plancher qui font un angle de 90° + 30° avec le plafond. Il est également proposé tout en maintenant le même angle de veine (va = 30°) d'incliner le plancher par rapport à l'axe de la turbine d'un angle vp de + ou − 25°.

Dans de tels aubages, les pertes secondaires sont importantes.

Pour diminuer les pertes, l'aubage directeur selon la présente invention est caractérisé en ce que les génératrices des aubes forment avec le plafond et avec la plancher un angle voisin de

$$90° + \frac{\theta}{2}$$

Cette disposition introduit évidemment une perte supplémentaire au plancher par rapport à la construction classique, mais réduit substantiellement la perte au plafond, de telle sorte que la somme des pertes au plafond et au plancher selon l'invention est inférieure à celle selon la construction classique.

La présente invention sera mieux comprise à la lumière de la description qui va suivre et des dessins annexés.

La figure 1 représente une coupe axiale d'une turbine à vapeur à veine divergente.

La figure 2 représente une vue de haut de l'aubage directeur.

La figure 3 représente une coupe selon la ligne A—B de la figure 2 dans le cas où la veine est cylindrique.

La figure 4 représente la même coupe dans le cas où la veine est divergente.

La figure 5 représente une coupe X—Y au niveau X1 Y1 de la figure 4.

La figure 6 représente un veine avec un aubage classique.

La figure 7 représente une veine avec un aubage selon l'invention.

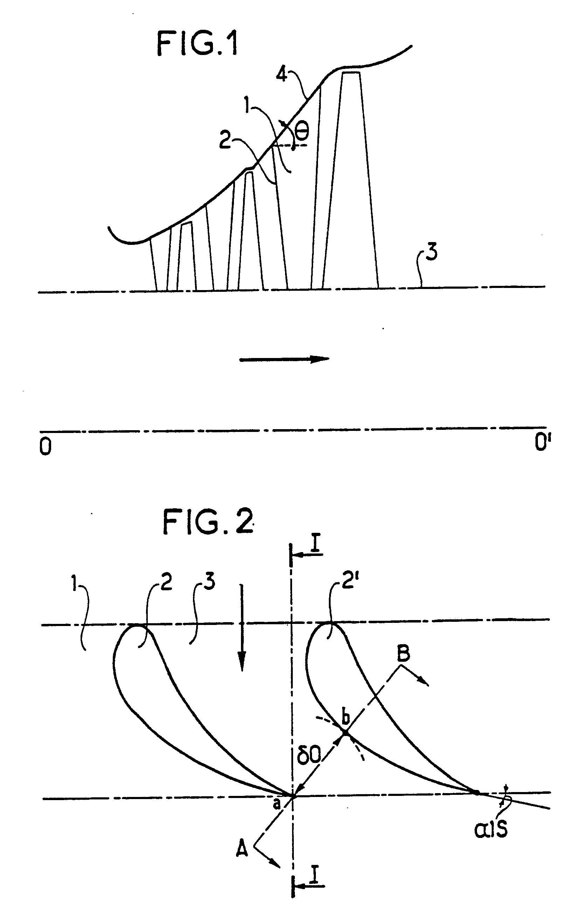

La figure 1 représente un turbe à vapeur dont les veines ont une forme divergente dans le sens du flux.

Cette turbine comporte une succession d'aubages fixes et d'aubages mobiles.

L'aubage directeur 1 du dernier étage comporte des aubes 2 disposées entre un plancher 3 cylindrique et un plafond conique 4 faisant un angle $\theta$ avec l'axe 0 0' de la turbine.

Cet angle $\theta$ est particulièrement important pour le dernier étage de la partie basse pression de la turbine. De plus, lorsqu'on désire utiliser des aubes mobiles terminales de grande hauteur, cette divergence est encore accentuée.

Dans les turbines connues, les valeurs de $\theta$ au droit de l'aubage directeur du dernier étage sont usuellement comprises entre 25° et 70°.

Sur la figure 2, on a représenté une vue de haut de l'aubage directeur 1 avec deux aubes consécutives 2, 2'.

La veine est limitée par l'intrados de l'aube 2, l'extrados de l'aube 2' et par la plafond et le plancher.

La distance minimale $\delta$ o entre les deux aubes voisines 2, 2' part de l'extrémité a du bord de fuite de l'aube 2 jusqu'à un point b de l'extrados de l'aube 2', le cercle de centre a et de rayon $\delta$ o étant tangent en b à l'extrados de l'aube 2'.

Le lieu l'espace sur toute la hauteur des aubes 2 et 2' des segments a, b donne la section minimmale de passage entre les deux aubes consécutives.

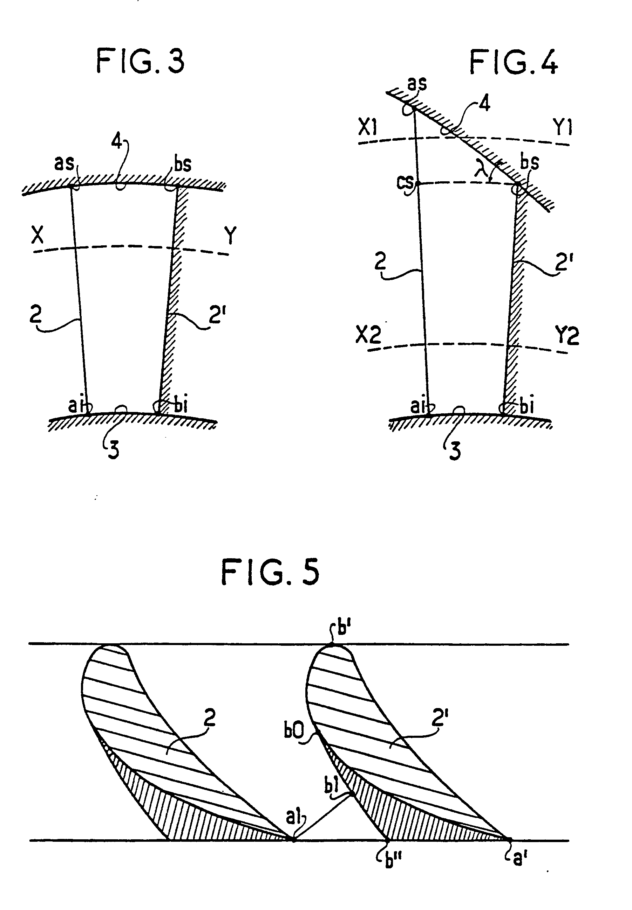

Lorsque l'angle $\theta$ est négligeable, cette section a sensiblement la forme d'un secteur d'anneau ai bi bs as (ai, bi étant sur le plancher et as, bs sur le plafond) compris entre la plancher 3, le plafond 4, le bord de fuite de l'aube 2 et l'extrados de l'aube 2' (voir figure 3).

Toute coupe cylindrique XY autour de l'axe de la turbine dans le cas de la figure 3 rencontre toutes ces aubes, quel que soit l'endroit où cette coupe est pratiquée sur la hauteur des aubes.

Lorsque l'angle $\theta$ est important, la section de passage minimale du flux a la forme d'un quadrilatère ai bi bs as (en négligeant les courbures de ai bi sur le plancher 3, et as bs sur le plafond 4) qui diffère essentiellement de la section représentée à la figure 3 par le triangle as bs cs, bs cs étant tracé parallèlement à ai bi (voir figure 4).

L'angle $\lambda$ = as bs cs du triangle as bs cs est d'autant plus voisin de l'angle $\theta$ que l'angle de sortie du jet a 1S avec le front de grille est petit.

En effet, plus a 1S est petit, plus la coupe passant par a et b se rapproche de la coupe I—I,

Toute coupe cylindrique XY axée sur l'axe de la turbine telle que X2 Y2 partiquée à une hauteur inférieure au niveau bs, cs, rencontre les aubes.

Par contre, toute coupe XY telle que X1, Y1 pratiquée sur la portion d'aube entre as et cs coupe l'aube 2 mais ne coupe plus l'aube 2' (voir cette coupe sur la figure 5).

La section de passage du flux est déterminée d'un côté par l'intrados de l'aube 2 et de l'autre côté par l'extrados de l'aube 2' dans une première partie b' bo, puis par le plafond de bo à b''.

En raison notamment de la surépaisseur bob'' a' (a' étant l'intersection du bord de fuite de l'aube 2' par le cylindre X1 Y1, avec X1 Y1 compris entre as et cs), il se produit de fortes pertes lors de la dénte de la vapeur traversant le triangle as bs cs.

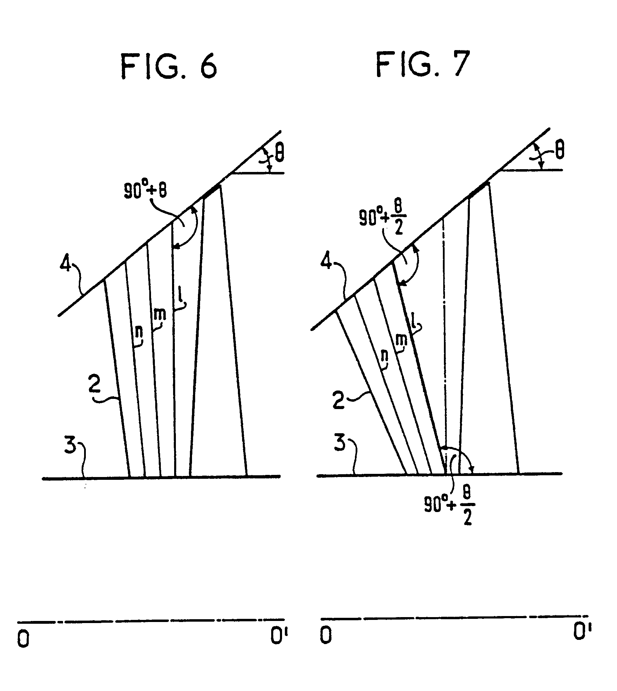

Sur la figure 6, on a représenté une aube classique. Les génératrices sensiblement rectilignes (l, m, n) formant les surfaces de l'extrados et de l'intrados sont orthogonales au plancher et un angle $\theta + 90°$ avec la plafond 4.

Sur la figure 7, l'aubage directeur est incliné de façon que les génératrices (l, m, n) forment avec le plafond 4 et la plancher 3 un angle voisin de

$$\frac{\theta}{2} + 90°.$$

## Revendication

Aubage directeur pour veines divergentes de turbine à vapeur comportant des aubes (2) disposées entre un plancher (3) parallèle à l'axe 0'0 de la turbine et un plafond (4) et dont l'extrados et l'intrados sont constitués par des génératrices (l, m, n) sensiblement rectilignes, l'angle de divergence $\theta$ de la veine étant comprise entre 25° et 70°, caractérisé en ce que lesdites génératrices (l, m, n) des aubes (2) forment avec le plafond (4) et avec le plancher (3), une angle voisin de

$$90° + \frac{\theta}{2}$$

## Patentanspruch

Leitschaufelanordnung für divergierende Strömungslinien in einer Dampfturbine, mit Leitschaufeln (2), die zwischen einem parallel zur Achse 0'0 der Turbine liegenden Boden (3) und einer Decke (4) angeordnet sind und deren Außen- und Innenflächen von im wesentlichen geradlinigen Mantellinien (l, m, n) gebildet werden, wobei der Divergenzwinkel $\theta$ der Strömungslinie zwischen 25 und 70° liegt, dadurch gekennzeichnet, daß diese Mantellinien (l, m, n) der Leitschaufeln (2) mit der Decke (4) und dem Boden (3) einen Winkel nahe $90° + \theta/2$ bilden.

## Claim

A guide blade set for diverging jet streams in a steam turbine, comprising blades (2) disposed between a floor (3) which is parallel to the axis 0'0 of the turbine and a ceiling (4) and having convex and concave surfaces constituted by substantially linear generatrix lines (l, m, n), wherein the angle of divergence $\theta$ of the jet stream is included between 25 and 70°, characterized in that said generatrix lines (l, m, n) of the blades (2) form an angle of about $90° + \theta/2$ with the ceiling (4) and with the floor (3).

# FIG.1

# FIG.2

# FIG. 3

# FIG. 4

# FIG. 5

# FIG. 6

# FIG. 7